# EUROPÄISCHE PATENTSCHRIFT

(11) **EP 1 200 502 B1**
(45) Veröffentlichungstag und Bekanntmachung des Hinweises auf die Patenterteilung: **09.03.2005**
(21) Anmeldenummer: 00944045.4
(22) Anmeldetag: 20.07.2000
(51) Int. Cl.: C08G 59/66, C08G 59/56, C07C 323/25

(54) **AMINHÄRTER FÜR EPOXIDHARZE**
AMINE HARDENER FOR EPOXY RESINS
DURCISSANT AMINIQUE POUR RESINES EPOXYDES

(30) Priorität: 28.07.1999 CH 138999
(43) Veröffentlichungstag der Anmeldung: 02.05.2002
(73) Patentinhaber: Huntsman Advanced Materials (Switzerland) GmbH, 4057 Basel (CH)
(72) Erfinder: FISCHER, Walter, CH-4153 Reinach (CH); GABUTTI, Claudio, Alexander, CH-4106 Therwil (CH); FRISCHINGER, Isabelle, F-68640 Riespach (FR); WIESENDANGER, Rolf, CH-4125 Riehen (CH)
(74) Vertreter: Dannappel, Hans-Jochen, Dr.
(86) Internationale Anmeldenummer: PCT/EP2000/006931
(87) Internationale Veröffentlichungsnummer: WO 2001/009221

(56) Entgegenhaltungen:
- EP-A- 0 273 170
- DE-A- 1 595 395
- US-A- 2 831 830
- US-A- 3 548 002
- US-A- 3 919 277
- PATENT ABSTRACTS OF JAPAN vol. 1995, no. 11, 26. Dezember 1995 (1995-12-26) & JP 07 228567 A (NIPPON SHOKUBAI CO LTD), 29. August 1995 (1995-08-29)
- DATABASE WPI Derwent Publications Ltd., London, GB; AN 1976-67628x XP002149954 & JP 51 082400 A (ASAHI DENKA KOGYO KK)

## Beschreibung

Die vorliegende Erfindung betrifft Polymercaptopolyamine, ein Verfahren zu deren Herstellung, Epoxidharz-Zusammensetzungen enthaltend solche Polymercaptopolyamine sowie die Verwendung dieser Zusammensetzungen.

PATENT ABSTRACT OF JAPAN vol. 1995, no. 11 (& JP-A-07228567) offenbart Verbindungen, die als Härter für Epoxyharze verwendet werden, die durch Reaktion eines aromatischen Amins, das an jedem der zwei Phenylringe eine NH₂-Gruppe trägt, mit einer Thiiran Komponente hergestellt werden.

Im U.S Patent Nr. 5,143,999 werden Mischungen aus Polyaminen und von Polyoxyalkylenglykolen abgeleiteten Dithiolen als Härter für Epoxidharze beschrieben. Die daraus hergestellten gehärteten Produkte zeichnen sich durch eine hohe Flexibilität und gute zähelastische Eigenschaften kombiniert mit guten Festigkeits- und Härte-Werten aus.

Aufgabe der vorliegenden Erfindung war es, Härtungsmittel für Epoxidharze bereitzustellen, die gehärtete Produkte mit verbesserter Chemikalienresistenz liefern.

Es wurde nun gefunden, daß bestimmte Polymercaptopolyamine schon bei tiefen Temperaturen hochreaktiv gegenüber Epoxidharzen sind und daß die daraus erhaltenen gehärteten Produkte sowohl eine verbesserte Chemikalienresistenz als auch eine erhöhte Witterungsbeständigkeit aufweisen.

Gegenstand der vorliegenden Erfindung sind Verbindungen der Formel la oder Ib, 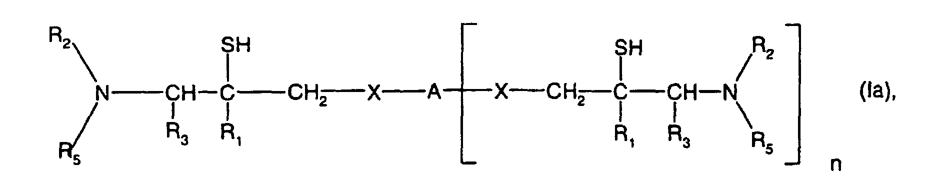 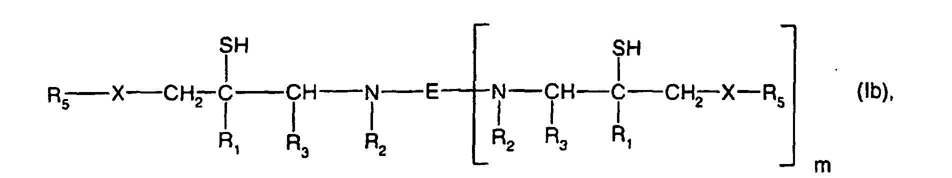 worin A einen (n + 1)-wertigen aliphatischen, cycloaliphatischen, araliphatischen oder aromatischen Rest darstellt und n eine ganze Zahl von 0 bis 5 ist,
E einen (m + 1)-wertigen aliphatischen, cycloaliphatischen, araliphatischen oder aromatischen Rest darstellt und m eine ganze Zahl von 0 bis 3 ist,
X für -O-, -COO-, oder -CHR₄- steht, wobei R₄ und R₃ zusammen eine Ethylengruppe bilden, R₁ und R₂ unabhängig voneinander Wasserstoff oder Methyl bedeuten,
R₃ Wasserstoff bedeutet oder R₃ und R₄ zusammen eine Ethylengruppe bilden,
und R₅ einen einwertigen aliphatischen, cycloaliphatischen, araliphatischen oder aromatischen Rest darstellt.

In der Formel la kann A grundsätzlich für jeden beliebigen ein- bis sechswertigen Rest eines Epoxids stehen. Bevorzugt sind zwei-, drei- und vierwertige Reste.

Beispiele für aliphatische Reste sind Ethylen, Propylen, Tetramethylen, Hexamethylen, Poly(oxyethylen), Poly(oxypropylen), Poly(oxytetramethylen), 2-Methyl-1,5-pentandiyl, 2,2,4-Trimethyl-1,6-hexandiyl, 2,4,4-Trimethyl-1,6-hexandiyl und die Reste von aliphatischen Alkoholen nach dem Entfernen der OH-Gruppen, wie beispielsweise die Reste des Trimethylolpropans, des Pentaerythrits und des Dipentarerythrits.

Cycloaliphatische Reste sind zum Beispiel Cyclopentyl, Cyclohexyl, 1,3-Cyclopentylen, 4-Methyl-1,3-cyclopentylen, 1,2-Cyclohexylen, 1,3-Cyclohexylen, 1,4-Cyclohexylen, 4-Methyl-1,3-cyclohexylen, 2,5-Norbornandiyl, 2,6-Norbomandiyl, 7,7-Dimethyl-2,5-Norbornandiyl, 7,7-Dimethyl-2,6-Norbornandiyl, Cyclohexan-1,3-dimethylen, Cyclohexan-1,4-dimethylen, 3-Methylen-3,5,5-trimethylcyclohexylen (Isophoron), Norbornan-2,5-dimethylen, Norbornan-2,6-dimethylen, 7,7-Dimethylnorbornan-2,5-dimethylen und 7,7-Dimethylnorbornan-2,6-dimethylen und die Reste von cycloaliphatischen Alkoholen nach dem Entfernen der OH-Gruppen, wie beispielsweise die Reste von hydriertem Bisphenol A und hydriertem Bisphenol F.

Geeignete araliphatische Reste sind beispielsweise Benzyl, die Reste von 1,2-, 1,3- und 1,4-Bis-(hydroxymethyl)benzol, die Reste von 1,2,3-, 1,2,4-, 1,2,5- und
1,3,5-Tris-(hydroxymethyl)benzol und die Reste von Bis-(hydroxymethyl)naphthalin. Beispiele für aromatische Reste sind Phenyl, Naphthyl, die Reste von Bisphenolen, wie Bisphenol A, Bisphenol F und Dihydroxybiphenyl, und die Reste von Phenol- und Kresolnovolaken.

Bevorzugt sind Verbindungen der Formel la, worin X -O- bedeutet und A für einen zweiwertigen Rest eines Bisphenols oder eines cycloaliphatischen Diols, für den Rest eines Phenol- oder Kresolnovolaks, für den zwei- bis vierwertigen Rest eines Isocyanat/Polyol-Addukts oder für den drei- bis sechswertigen Rest eines tri- bis hexafunktionellen aliphatischen Polyols steht.

Besonders bevorzugt sind Verbindungen der Formel la, worin X -O- bedeutet und A für einen zweiwertigen Rest der Formeln 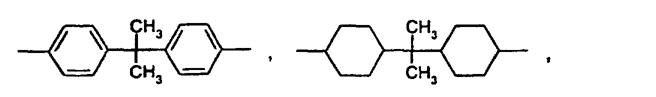 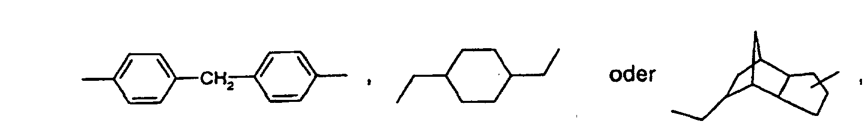 für den Rest eines Phenol- oder Kresolnovolaks, für einen dreiwertigen Rest der Formeln 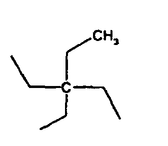 oder 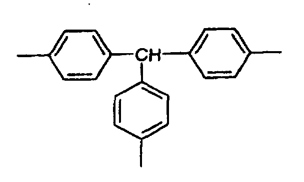 oder für den vierwertigen Rest der Formel 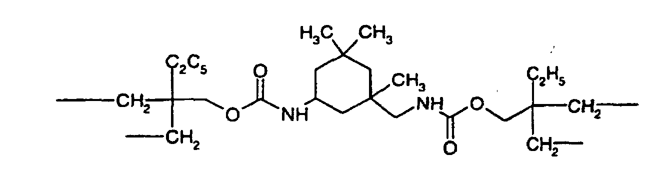 steht.

R₅ steht in der Formel Ia und Ib vorzugsweise für unsubstituiertes oder mit einer oder mehreren Aminogruppen, Hydroxylgruppen, C₁-C₈-Alkoxygruppen oder Halogenatomen substituiertes C₁-C₂₀-Alkyl, C₅-C₁₂-Cycloalkyl, C₆-C₁₀-Aryl oder C₇-C₁₂-Aralkyl.

Geeignete Alkylgruppen als R₅ sind zum Beispiel Methyl, Ethyl, Isopropyl, n-Propyl, n-Butyl, Isobutyl, sek.-Butyl, tert.-Butyl sowie die verschiedenen isomeren Pentyl-, Hexyl-, Heptyl-, Octyl-, Nonyl-, Decyl-, Undecyl-, Dodecyl-, Tridecyl-, Tetradecyl-, Pentadecyl-, Hexadecyl-, Heptadecyl- und Octadecylgruppen.

Bei Cycloalkyl handelt es sich bevorzugt um C₅-C₈-Cycloalkyl, besonders um C₅- oder C₆-Cycloalkyl. Einige Beispiele sind Cyclopentyl, Methylcyclopentyl, Cyclohexyl, Cycloheptyl und Cyclooctyl.

Aralkyl enthält bevorzugt 7 bis 12 C-Atome und besonders bevorzugt 7 bis 10 C-Atome. Es kann sich zum Beispiel um Benzyl, Phenethyl, 3-Phenylpropyl, α-Methylbenzyl, 4-Phenylbutyl und α,α-Dimethylbenzyl handeln.

Arylgruppen sind beispielsweise Phenyl, Tolyl, Mesityl, Isityl, Naphthyl und Anthryl.

Bevorzugt sind Verbindungen der Formel la und Ib, worin R₅ für C₂-C₁₀-Alkyl, C₂-C₁₀-Aminoalkyl; Phenyl, Benzyl, Cyclohexyl oder für einen Rest der Formel H₂N-Z-CH₂-NH- steht, worin Z einen zweiwertigen cycloaliphatischen, araliphatischen oder aromatischen Rest oder einen Rest der Formel -(CH₂CH₂NH)ₖ-CH₂- darstellt, worin k 2 oder 3 bedeutet.

Geeignete Reste Z sind beispielsweise die oben für A angegebenen zweiwertigen Reste.

Besonders bevorzugt sind Verbindungen der Formel Ia und Ib, worin R₅ für n-Butyl, n-Octyl, Cyclohexyl, Benzyl, 2-Aminoethyl, 4-(Aminomethyl)pentyl, 5-Amino-2-methylpentyl, 3-Dimethylaminopropyl, 3-Methylaminopropyl, 4-Aminocyclohexyl oder für einen Rest der Formeln -CH₂CH₂NHCH₂CH₂NH₂, 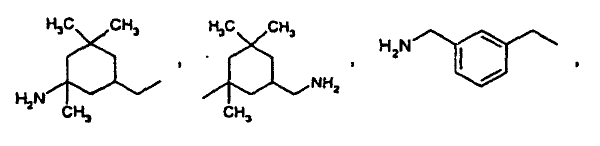 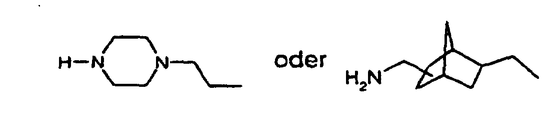 steht.

Bevorzugt sind außerdem Verbindungen der Formel Ia oder Ib, worin X für O- steht und R₁ und R₃ Wasserstoff bedeuten.

Die Verbindungen der Formel la können nach bekannten-Methoden aus den Epoxidverbindungen der Formel IIa hergestellt werden, worin A, X, R₁, R₃ und n die oben angegebene Bedeutung haben:

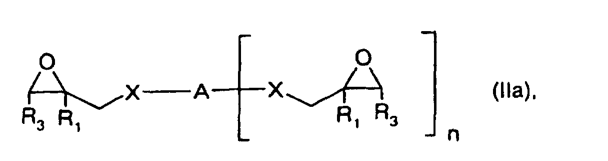

Dabei wird die Epoxidverbindung der Formel IIa in einem ersten Reaktionsschritt durch Umsetzung mit Thioharnstoff oder einem Alkali- oder Ammoniumthiocyanat, vorzugsweise Kaliumthiocyanat, in das Episulfid der Formel IIIa überführt

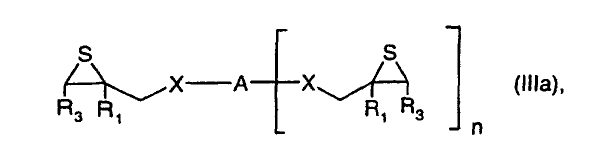

Thioharnstoff bzw. Thiocyanat wird dabei zweckmäßig in einer solchen Menge eingesetzt, daß auf ein Epoxidäquivalent 0,8 bis 1,2 Äquivalente Schwefel entfallen.
Die Reaktion kann in aprotischen oder protischen organischen Lösungsmitteln oder Gemischen davon durchgeführt werden. Bevorzugt sind Alkohole, wie Methanol oder Ethanol, und aromatische Kohlenwasserstoffe, wie Toluol und Xylol. Die Zugabe von Cosolventien, wie Ether oder Carbonsäuren, kann die Reaktion beschleunigen.
Die Reaktion kann sowohl bei Raumtemperatur als auch bei erhöhter Temperatur durchgeführt werden; die bevorzugte Reaktionstemperatur liegt zwischen 60 und 100 °C. Das Episulfid der Formel IIIa kann isoliert werden durch Abtrennung der Nebenprodukte mittels Filtration, Extraktion, Phasentrennung und anschließendes Eindampfen des Lösungsmittels.
Es ist aber auch möglich, das Episulfid der Formel IIIa als Rohprodukt in Lösung ohne Abtrennung der Nebenprodukte direkt weiterzuverarbeiten.

Das Episulfid der Formel IIIa wird dann in einem aprotischen oder protischen organischen Lösungsmittel gelöst und unter Inertgas (Argon oder Stickstoff) mit dem Amin R₅-NH-R₂ umgesetzt. Die Menge des Amins wird dabei vorzugsweise so gewählt, daß auf eine Episulfidgruppe 1-10 NH-Gruppen entfallen. Bevorzugte Lösungsmittel sind Alkohole (z.B. Methanol, Ethanol, t-Butanol) und aromatische Kohlenwasserstoffe, wie Toluol oder Xylol. Auch das Amin R₁-NH₂ wird vorzugsweise als Lösung in einem der oben erwähnten organischen Lösungsmittel eingesetzt.

Die Umsetzung wird zweckmäßig bei erhöhter Temperatur durchgeführt, vorzugsweise bei 40 °C - 120 °C.
Die erfindungsgemäßen Verbindungen der Formel la können isoliert werden, indem das Lösungsmittel unter vermindertem Druck abdestilliert wird. Der Überschuss an Amin R₅-NH-R₂ kann dann bei erhöhter Temperatur ebenfalls destillativ entfernt werden. In einer besonderen Ausführungsform der Erfindung wird das Amin R₅-NH-R₂ als Co-Härter eingesetzt; in diesem Fall ist eine Trennung von Produkt der Formel la und Amin R₅-NH-R₂ nicht notwendig, sondern das Reaktionsprodukt kann ohne weitere Aufarbeitung als Härter für Epoxidharze eingesetzt werden. Dieses Vorgehen empfiehlt sich insbesondere bei Verwendung von Di- oder Polyaminen.

Gegenstand der vorliegenden Erfindung ist somit auch ein Verfahren zur Herstellung von Verbindungen der Formel la durch Reaktion einer Verbindung der Formel IIa 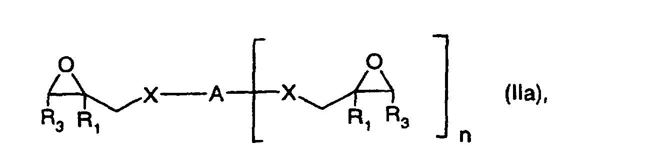 worin A, X, R₁, R₃ und n die oben angegebene Bedeutung haben,
mit Thioharnstoff oder einem Thiocyanat und anschließender Umsetzung des so erhaltenen Episulfids mit-einem Amin der Formel R₅-NH-R₂, worin R₅ und R₂ die oben angegebene Bedeutung haben.

Die Verbindungen der Formel Ib lassen sich analog aus den entsprechenden Epoxidverbindungen der Formel IIb herstellen.

Einen weiteren Erfindungsgegenstand bildet daher ein Verfahren zur Herstellung von Verbindungen der Formel Ib durch Reaktion einer Verbindung der Formel IIb 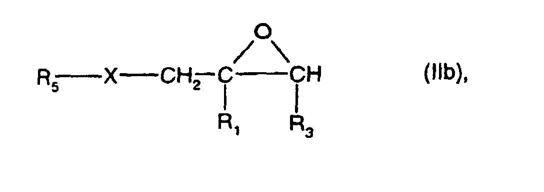 worin X, R₁, R₃ und R₅ die oben angegebene Bedeutung haben,
mit Thioharnstoff oder einem Thiocyanat und anschließender Umsetzung des so erhaltenen Episulfids mit einem Polyamin der Formel E-(NHR₂)ₘ₊₁, worin E R₂ und m die oben angegebene Bedeutung haben.

Episulfide können zum Beispiel auch aus den entsprechenden Epoxiden durch Umsetzung mit Triphenylphosphinsulfid synthetisiert werden.

Außerdem können Episulfide nach bekannten Methoden direkt aus den entprechenden Alkenen hergestellt werden, beispielsweise durch Umsetzung mit m-Chlorperbenzoesäure und anschließender Reaktion mit Thioharnstoff in Gegenwart von H₂SO₄, durch Umsetzung mit Propylensulfid in Gegenwart von Rhodium-Katalysatoren sowie durch Reaktion mit (Diethoxyphosphoryl)sulfenytchlorid, (Diethoxythiophosphoryl)sutfenylbromid, Thiobenzophenone S-oxid oder Bis(trimethylsilyl)sulfid.

Wie eingangs erwähnt, eignen sich die erfindungsgemäßen Polymercaptopolyamine insbesondere als Härter für Epoxidharze.

Einen weiteren Erfindungsgegenstand bildet eine Zusammensetzung enthaltend
(A) ein Epoxidharz mit durchschnittlich mehr als einer 1,2-Epoxidgruppe pro Molekül und
(B) eine Verbindung der Formel Ia oder Ib.

Zur Herstellung der erfindungsgemäßen Zusammensetzungen eignen sich als Komponente A die in der Epoxidharztechnik üblichen Epoxidharze. Beispiele für Epoxidharze sind:
I) Polyglycidyl- und Poly-(β-methylglycidyl)-ester, erhältlich durch Umsetzung einer Verbindung mit mindestens zwei Carboxylgruppen im Molekül und Epichlorhydrin bzw. β-Methylepichlorhydrin. Die Umsetzung erfolgt zweckmäßig in der Gegenwart von Basen.
   Als Verbindung mit mindestens zwei Carboxylgruppen im Molekül können aliphatische Polycarbonsäuren verwendet werden. Beispiele für solche Polycarbonsäuren sind Oxalsäure, Bernsteinsäure, Glutarsäure, Adipinsäure, Pimelinsäure, Korksäure, Azelainsäure oder dimerisierte bzw. trimerisierte Linolsäure.
   Es können aber auch cycloaliphatische Polycarbonsäuren eingesetzt werden, wie beispielsweise Tetrahydrophthalsäure, 4-Methyltetrahydrophthalsäure, Hexahydrophthalsäure oder 4-Methylhexahydrophthalsäure.
   Weiterhin können aromatische Polycarbonsäuren Verwendung finden, wie beispielsweise Phthalsäure, Isophthalsäure oder Terephthalsäure.
II) Polyglycidyl-oder Poly-(β-methylglycidyl)-ether, erhältlich durch Umsetzung einer Verbindung mit mindestens zwei freien alkoholischen Hydroxygruppen und/oder phenolischen Hydroxygruppen mit Epichlorhydrin oder β-Methylepichlorhydrin unter alkalischen Bedingungen oder in Anwesenheit eines sauren Katalysators mit anschließender Alkalibehandlung.
   Die Glycidylether dieses Typs leiten sich beispielsweise von acyclischen Alkoholen ab, z.B. von Ethylenglykol, Diethylenglykol oder höheren Poly-(oxyethylen)glykolen, Propan-1,2-diol oder Poly-(oxypropylen)-glykolen, Propan-1,3-diol, Butan-1,4-diol, Poly-(oxytetrarnethylen)-glykolen, Pentan-1,5-diol, Hexan-1,6-diol, Hexan-2,4,6-triol, Glycerin, 1,1,1 -Trimethylolpropan, Pentaerythrit, Sorbit, sowie von Polyepichlorhydrinen.
   Weitere Glycidylether dieses Typs leiten sich ab von cycloaliphatischen Alkoholen, wie 1,4-Cyclohexandimethanol, Bis-(4-hydroxycyclohexyl)-methan oder 2,2-Bis-(4-hydroxycyclohexyl)-propan, oder von Alkoholen, die aromatische Gruppen und/oder weitere funktionelle Gruppen enthalten, wie N,N-Bis-(2-hydroxyethyl)-anilin oder p,p'-Bis-(2-hydroxyethylamino)-diphenylmethan.
   Die Glycidylether können auch auf einkerningen Phenolen, wie beispielsweise Resorcin oder Hydrochinon, oder auf mehrkernigen Phenolen, wie beispielsweise Bis-(4-hydroxyphenyl)-methan, 4,4'-Dihydroxybiphenyl, Bis-(4-hydroxyphenyl)-sulfon, 1,1,2,2-Tetrakis-(4-hydroxyphenyl)-ethan, 2,2-Bis-(4-hydroxyphenyl)-propan oder 2,2-Bis-(3,5-dibrom-4-hydroxyphenyl)-propan, basieren.
   Weitere geeignete Hydroxyverbindungen zur Herstellung von Glycidylethern sind Novolake, erhältlich durch Kondensation von Aldehyden, wie Formaldehyd, Acetaldehyd, Chloral oder Furfuraldehyd, mit Phenolen oder Bisphenolen, die unsubstituiert oder mit Chloratomen oder C₁-C₉-Alkylgruppen substituiert sind, wie beispielsweise Phenol, 4-Chlorphenol, 2-Methylphenol oder 4-tert.-Butylphenol.
III) Poly-(N-glycidyl)-verbindungen, erhältlich durch Dehydrochlorierung der Reaktionsprodukte von Epichlorhydrin mit Aminen, die mindestens zwei Aminwasserstoffatome enthalten. Bei diesen Aminen handelt es sich zum Beispiel um Anilin, n-Butylamin, Bis-(4-aminophenyl)-methan, m-Xylylendiamin oder Bis-(4-methylaminophenyl)-methan.
   Zu den Poly-(N-glycidyl)-verbindungen zählen aber auch Triglycidylisocyanurat, N,N'-Di-glycidylderivate von Cycloatkylenhamstoffen, wie Ethylenharnstoff oder 1,3-Propylenharnstoff, und Diglycidylderivate von Hydantoinen, wie von 5,5-Dimethylhydantoin.
IV) Poly-(S-glycidyl)-verbindungen, beispielsweise Di-S-glycidylderivate, die sich von Dithiolen, wie beispielsweise Ethan-1,2-dithiol oder Bis-(4-mercaptomethylphenyl)-ether, ableiten.
V) Cycloaliphatische Epoxidharze, wie beispielsweise Bis-(2,3-epoxycyclopentyl)-ether, 2,3-Epoxycyclopentylglycidylether, 1,2-Bis-(2,3-epoxycyclopentyloxy)-ethan oder 3,4-Epoxycyclohexylmethyl-3',4'-epoxycyclohexancarboxylat.

Es lassen sich aber auch Epoxidharze verwenden, bei denen die 1,2-Epoxidgruppen an unterschiedliche Heteroatome bzw. funktionelle Gruppen gebunden sind; zu diesen Verbindungen zählen beispielsweise das N,N,O-Triglycidylderivat des 4-Aminophenols, der Glycidylether-glycidylester der Salicylsäure, N-Glycidyl-N'-(2-glycidyloxypropyl)-5,5-dimethylhydantoin oder 2-Glycidyloxy-1,3-bis-(5,5-dimethyl-1-glycidylhydantoin-3-yl)-propan.

Bevorzugt verwendet man zur Herstellung der erfindungsgemäßen Epoxidharzzusammensetzungen einen flüssigen oder festen Polyglycidylether oder -ester, insbesondere einen flüssigen oder festen Bisphenoldiglycidylether oder einen festen oder flüssigen Diglycidylester einer cycloaliphatischen oder aromatischen Dicarbonsäure, oder ein cycloaliphatisches Epoxidharz. Es können auch Gemische von Epoxidharzen verwendet werden.

Als feste Polyglycidylether und -ester kommen Verbindungen mit Schmelzpunkten oberhalb Raumtemperatur bis etwa 250°C in Betracht. Bevorzugt liegen die Schmelzpunkte der festen Verbindungen im Bereich von 50 bis 150°C. Solche festen Verbindungen sind bekannt und zum Teil im Handel erhältlich. Als feste Polyglycidylether und -ester können auch die durch Vorverlängerung von flüssigen Polyglycidylethern und -estern erhaltenen Advancement-Produkte verwendet werden.

Insbesondere enthalten die erfindungsgemäßen Epoxidharzzusammensetzungen einen flüssigen Polyglycidylether oder -ester.

Besonders bevorzugt als Komponente A sind Bisphenol A-diglycidylether, Bisphenol F-diglycidylether, Gemische aus Bisphenol A-diglycidylether und Bisphenol F-diglycidylether, Epoxyurethane, aliphatische Epoxidharze wie Trimethylolpropantriglycidylether sowie cycloaliphatische Epoxidharze wie Hexahydrophthalsäurediglycidylester.

Die erfindungsgemäßen Polymercaptopolyamine können vorteilhaft in Kombination mit anderen Epoxid-Härtern, insbesondere mit den üblichen Aminhärtern, eingesetzt werden.

Einen weiteren Erfindungsgegenstand bildet daher eine Zusammensetzung enthaltend
(A) ein Epoxidharz,
(B) eine Verbindung der Formel la oder Ib und
(C) ein Polyamin.

Beispiele für geeignete Polyamine C sind aliphatische, cycloaliphatische, aromatische und heterocyclische Amine, wie Bis(4-aminophenyl)methan, Anilin-Formaldehyd-Harze, Benzylamin, n-Octylamin, Propan-1,3-diamin, 2,2-Dimethyl-1,3-propandiamin (Neopentandiamin), Hexamethylendiamin, Diethylentriamin, Bis(3-aminopropyl)amin, N,N-Bis-(3-aminopropyl)methylamin, Triethylentetramin, Tetraethylenpentamin, Pentaethylenhexamin, 2,2,4-Trimethylhexan-1,6-diamin, m-Xylylendiamin, 1,2- und 1,4-Diaminocyclohexan, Bis(4-aminocyclohexyl)methan, Bis(4-amino-3-methylcyclohexyl)methan, 2,2-Bis(4-aminocyclohexyl)propan und 3-Aminomethyl-3,5,5-trimethylcyclohexylamin (Isophorondiamin), Polyaminoimidazoline sowie Polyaminoamide, wie beispielsweise solche aus aliphatischen Polyaminen und dimerisierten oder trimerisierten Fettsäuren. Geeignete Amine(C) sind auch die als Jeffamine bekannten Polyoxyalkylenamine der Firma Texaco, wie z.B. Jeffamine EDR148, D230, D400 oder T403.

Weitere geeignete Polyamine (C) sind 1,14-Diamino-4,11-dioxatetradecan, Dipropylentriamin, 2-Methyl-1,5-pentandiamin, N,N'-Dicyclohexyl-1,6-hexandiamin, N,N'-Dimethyl-1,3-diaminopropan, N,N'-Diethyl-1,3-diaminopropan, N,N-Dimethyl-1,3-diaminopropan, sekundäre Polyoxypropylendi- und -triamine, 2,5-Diamino-2,5-dimethylhexan, Bis-(amino-methyl)tricyclopentadien, m-Aminobenzylamin, 1,8-Diamino-p-menthan, Bis- (4-amino-3,5-dimethylcyclohexyl)methan, 1,3-Bis(aminomethyl)cyclohexan, Dipentylamin, Bis(4-amino3,5-diethylphenyl)-methan, 3,5-Diethyltoiuol-2,4-diamin und 3,5-Diethyltoluol-2,6-diamin.

Bevorzugt als Komponente A der erfindungsgemässen Stoffgemische sind cycloaliphatische und aliphatische Amine, insbesondere die zur Herstellung der erfindungsgemäßen Polymercaptopolyamine verwendeten Amine der Formeln R₅-NH-R₂ und E-(NHR₂)ₘ₊₁.

Das Mengenverhältnis der Komponenten A und B und gegebenenfalls C kann in den erfindungsgemäßen Zusammensetzungen in weiten Bereichen variieren. Das optimale Verhältnis ist u.a. abhängig vom Amintyp und kann vom Fachmann leicht ermittelt werden.

Die Komponenten B und gegebenfalls C werden vorzugsweise in solchen Mengen eingesetzt, daß die Summe der Amin- und Mercaptanäquivalente 0,5 bis 2,0, besonders bevorzugt 0,8 bis 1,5 und insbesondere bevorzugt 0,9 bis 1,2 Äquivalente, bezogen auf ein Epoxidäquivalent, beträgt.

Die erfindungsgemäßen Zusammensetzungen können gegebenenfalls Beschleuniger, wie zum Beispiel tertiäre Amine oder Imidazole, enthalten.

Weiterhin können die härtbaren Mischungen Zähigkeitsvermittler ("Toughener") enthalten, wie zum Beispiel Core/Shell-Polymere oder die dem Fachmann als "Rubber Toughener" bekannten Elastomere oder Elastomere enthaltende Pfropfpolymere.
Geeignete Zähigkeitsvermittler sind beispielsweise in der EP-A-449 776 beschrieben.

Außerdem können die härtbaren Mischungen Füllstoffe enthalten, wie beispielsweise Metallpulver, Holzmehl, Glaspulver, Glaskugeln, Halbmetall- und Metalloxide, wie zum Beispiel SiO₂ (Aerosile, Quarz, Quarzmehl, Quarzgutmehl), Korund und Titanoxid, Halbmetall- und Metallnitride, wie zum Beispiel Siliziumnitrid, Bornitrid und Aluminiumnitrid, Halbmetall- und Metallcarbide (SiC), Metallcarbonate (Dolomit, Kreide, CaCO₃), Metallsulfate (Baryt, Gips), Gesteinsmehle und natürliche oder synthetische Mineralien hauptsächlich aus der Silikatreihe, wie zum Beispiel Zeolithe (insbesondere Molekularsiebe) Talkum, Glimmer, Kaolin,Wollastonit, Bentonit und andere.

Neben den oben erwähnten Additiven können die härtbaren Gemische weitere übliche Zusatzstoffe enthalten, wie z.B. Antioxidantien, Lichtschutzmittel, Weichmacher, Farbstoffe, Pigmente, Thixotropiemittel, Zähigkeitsverbesserer, Entschäumer, Antistatika, Gleitmittel und Entformungshilfsmittel.

Die Härtung der erfindungsgemäßen Epoxidharzzusammensetzungen zu Formkörpern, Beschichtungen oder dergleichen erfolgt in für die Epoxidharztechnik üblicher Weise, wie sie beispielsweise im "Handbook of Epoxy Resins", 1967, von H. Lee und K.Neville beschrieben wird.

Besonders hervorzuheben ist die hohe Reaktivität der erfindungsgemäßen Polymercaptopolyamine gegenüber Epoxidharzen schon bei tiefen Temperaturen (-5 °C bis 25 °C).
Die härtbaren Mischungen weisen nur eine geringe Neigung zur Carbonatisierung (Trübung) auf.
Die gehärteten Produkte zeichnen sich durch eine überraschend hohe Chemikalienresistenz und Witterungsbeständigkeit aus.

Die durch Härtung einer erfindungsgemäßen Zusammensetzung erhältlichen vernetzten Produkte stellen einen weiteren Erfindungsgegenstand dar.

Die erfindungsgemäßen Zusammensetzungen eignen sich ausgezeichnet als Beschichtungsmittel, Klebstoff, Bindemittel für Verbundwerkstoffe oder Gießharz zur Herstellung von Formkörpern.

### Beispiele:

### I. Herstellung der Verbindungen der Formel I

a) Allgemeine Vorschrift zur Herstellung der Polyepisulfide:
   Das Polyepoxid der Formel II wird in der 0,5- bis 5-fachen Menge Lösungsmittel gelöst und unter Stickstoff mit Thioharnstoff bzw. Alkali- oder Ammoniumthiocyanat (0,8-1,2 Äquivalente Schwefel pro Epoxidäquivalent) solange bei 60-100 °C gerührt, bis der Epoxidgehalt auf nahezu Null gefallen ist.
   Nach Abtrennung der Nebenprodukte mittels Filtration, Extraktion oder Phasentrennung wird das Polyepisulfid durch Eindampfen des Lösungsmittels isoliert.
b) Allgemeine Vorschrift zur Herstellung der Polymercaptopolyamine:
   Das Polyepisulfid wird in der 0,5- bis 5-fachen Menge Lösungsmittel gelöst und unter Stickstoff unter starkem Rühren mit dem Amin vereinigt, welches ebenfalls in der 0,5- bis 5-fachen Menge Lösungsmittel gelöst ist. Die Menge des Amins wird dabei so gewählt, daß auf eine Episulfidgruppe 1-10 NH₂-Gruppen entfallen. Nach 0,2- bis 3-stündigem Rühren bei 60-100 °C wird das Lösungsmittel unter vermindertem Druck abdestilliert. Zur Isolierung des Polymercaptopolyamins der Formel I wird der Überschuss an Amin-Reagens durch Vakuumdestillation bei erhöhter Temperatur entfernt.
   In einer Ausführungsform der Erfindung wird auf die Entfernung des überschüssigen Amins verzichtet, und das Gemisch aus dem Amin R₁-NH₂ und dem Polymercaptopolyamin der Formel I wird als Härter für Epoxidharze eingesetzt.

Nach der oben angegebenen Arbeitsvorschrift werden aus folgenden Diaminen R₁-NH₂ und Epoxiden der Formel II erfindungsgemäße Polymercaptopolyamine hergestellt (Beispiele 1.1-1.19):
- BA:: n-Butylamin
- OA:: n-Octylamin
- CYA:: Cyclohexylamin
- BZA:: Benzylamin
- MBA:: Methylbutylamin
- DMDP:: N,N-Dimethyl-1,3-diaminopropan
- MDP:: N-Methyl-1,3-diaminopropan
- DACY:: 1,2-Diaminocyclohexan
- AEP:: N-2-Aminoethylpiperazin
- DETA:: Diethylentriamin
- IPD:: Isophorondiamin
- MXDA:: meta-Xylylendiamin
- DYTEK-A:: 1,5-Diamino-2-methylpentan
- NBDA:: Isomerengemisch aus 2,5- und 2,6-Bis(aminomethyl)norbornan
- EDA:: Ethylendiamin
- Epoxid 1:: flüssiger Bisphenol A-diglycidylether mit einem Epoxidgehalt von 5,25-5,4 val/kg
- Epoxid 2:: flüssiges Gemisch aus Bisphenol A-diglycidylether und Bisphenol F-diglycidylether mit einem Epoxidgehalt von 5,5-5,8 val/kg
- Epoxid 3:: 1,4-Bis(hydroxymethyl)cyclohexandiglycidylether
- Epoxid 4:: Epoxyphenolnovolak mit einem Epoxidgehalt von 5,6-5,8 val/kg
- Epoxid 5:: Diglycidylether von hydriertem Bisphenol A
- Epoxid 6:: Tetraglycidylether der Formel

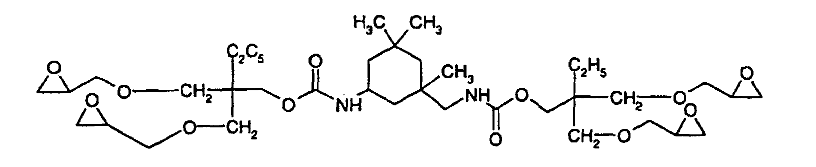

- Epoxid 7:: Bisphenol A-di-(β-methylglycidyl)ether
- Epoxid 8:: Trimethylolpropantriglycidylether
- Epoxid 9:: Hexahydrophthalsäurediglycidylester (Epoxidzahl: 5,6-6,2 val/kg)
- Epoxid 10:: Phenylglycidylether

Die Reaktionsbedingungen und die Eigenschaften der Endprodukte sind in Tabelle 1 angegeben.

**Tabelle 1:**

| Beispiel | Ausgangsprodukte | | Molverhältnis Episulfid/Amin | T/°C | Viskosität [mPa · s] | Aminwert [Äquivalent/kg] |
|---|---|---|---|---|---|---|
| | Amin | Epoxid | | | | |
| I.1 | DETA | Epoxid 1 | 1 : 5 | 100 | 1900^{*)} | 10,5 |
| I.2 | DETA | Epoxid 1 | 1 : 5 | 100 | >50000 | 7,1 |
| I.3 | IPD | Epoxid 1 | 1 : 5 | 100 | 5300 ^{*)} | 7,6 |
| I.4 | MXDA | Epoxid 1 | 1 : 5 | 60 | 2030^{*)} | 9,6 |
| I.5 | MXDA | Epoxid 1 | 1 : 5 | 60 | >50000 | - |
| I.6 | MXDA | Epoxid 1 | 1 : 4 | 60 | 9000 ^{*)} | 8,5 |
| I.7 | MXDA | Epoxid 2 | 1 : 5 | 55 | 2200 ^{*)} | 9,2 |
| I.8 | MXDA | Epoxid 2 | 1 : 4 | 55 | 4400 ^{*)} | 8,1 |
| I.9 | MXDA | Epoxid 2 | 1 : 3 | 55 | 50000 ^{*)} | 7,6 |
| I.10 | IPD | Epoxid 2 | 1 : 5 | 100 | 27000^{*)} | 7,8 |
| I.11 | I PD | Epoxid 2 | 1 : 3 | 100 | >50000^{*)} | 6,3 |
| I.12 | DETA | Epoxid 2 | 1 : 5 | 100 | 2000 ^{*)} | - |
| I.13 | DYTEK-A | Epoxid 2 | 1 : 5 | 100 | 1600^{*)} | 10,0 |
| I.14 | NBDA | Epoxid 2 | 1 : 5 | 60 | 13500^{*)} | 8,4 |
| I.15 | EDA | Epoxid 2 | 1 : 5 | 100 | >50000 | - |
| I.16 | MXDA | Epoxid 3 | 1 : 5 | 60 | 300^{*)} | 9,8 |
| I.17 | MXDA | Epoxid 3 | 1 : 4 | 60 | 500^{*)} | 8,8 |
| I.18 | IPD | Epoxid 3 | 1 : 4 | 60 | 970^{*)} | - |
| I.19 | DETA | Epoxid 3 | 1 : 5 | 100 | >50000 | - |
| I.20 | BA | Epoxid 2 | 1 : 10 | 77 | >60000 | 2,6 |
| I.21 | CYA | Epoxid 2 | 1 : 10 | 100 | >60000 | 3,3 |
| I.22 | BZA | Epoxid 2 | 1 : 10 | 100 | >60000 | 2,8 |
| I.23 | OA | Epoxid 2 | 1 : 10 | 90 | >60000 | |
| I.24 | CYA | Epoxid 4 | 1 : 10 | 100 | >60000 | 4,1 |
| I.25 | BZA | Epoxid 4 | 1 : 10 | 100 | >60000 | 3,6 |
| I.26 | BA | Epoxid 3 | 1 : 10 | 80 | 3000-7000 | 2,9 |
| I.27 | CYA | Epoxid 3 | 1 : 10 | 100 | >60000 | 3,1 |
| I.28 | BZA | Epoxid 3 | 1 : 10 | 100 | 10200 | 3,0 |
| I.29 | OA | Epoxid 3 | 1 : 10 | 100 | | |
| I.30 | BA | Epoxid 5 | 1 : 10 | 75 | >128000 | 2,15 |
| I.31 | MBA | Epoxid 5 | 1 : 10 | 75 | 13440 | - |
| I.32 | BA | Epoxid 6 | 1 : 20 | 75 | >128000^{**)} | 2,04 |
| I.33 | MBA | Epoxid 6 | 1 : 20 | 60 | 12800 | - |
| I.34 | IPD | Epoxid 6 | 1 : 20 | 60 | >128000^{**)} | 7,83 |
| I.35 | DMDP | Epoxid 3 | 1 : 10 | 70 | 1400 | 7,42 |
| I.36 | MDP | Epoxid 3 | 1 : 10 | 65 | 43520 | 6,17 |
| I.37 | DACY | Epoxid 3 | 1 : 5 | 65 | 840 | 10,71 |
| I.38 | AEP | Epoxid 3 | 1 : 5 | 65 | 400 | 10,45 |
| I.39 | MBA | Epoxid 3 | 1 : 2 | 67 | 1040 | - |
| I.40 | BA | Epoxid 7 | 1 : 10 | 70 | >128000 | 3,10 |
| I.41 | DACY | Epoxid 3 | 1 : 2 | 75 | 87040 | 6,34 |
| I.42 | AEP | Epoxid 3 | 1 : 2 | 77 | 11520 | 6,72 |
| I.43 | MBA | Epoxid 6 | 1 : 15 | 80 | 1160 | - |
| I.44 | MBA | Epoxid 8 | 1 : 3 | 70 | >128000 | - |
| I.45 | IPD | Epoxid 10 | 2 : 1 | 75 | >128000 | 2,83 |
| I.46 | BA | Epoxid 10 | 1 : 5 | 78 | 3040 | 2,75 |

| | | | | | | |
|---|---|---|---|---|---|---|
| ^{*)} Gemisch aus Amin R₁-NH₂ und Polymercaptopolyamin; auf destillative Entfernung des Überschusses an R₁-NH₂ ist verzichtet worden. | | | | | | |
| ^{**)} praktisch geliert | | | | | | |

### Anwendungsbeispiele

### II.1 Polymercaptopolyamin als Härter für Epoxidharze

100 g eines flüssigen Bisphenol A-diglycidylethers mit einem Epoxidgehalt von 5,25-5,4 val/kg werden mit 28 g des Polymercaptopolyamins aus Beispiel 1.4 bei 20 °C vermischt. Das Gemisch wird mittels Rakel auf Glasplatten oder Stahlplatten aufgezogen (Schichtdicke: 0,2 mm) und 10 d bei 20 °C ausgehärtet.
Zum Vergleich werden 100 g des gleichen Epoxidharzes mit 20 g eines handelsüblichen Aminhärters (DETA) unter den gleichen Bedingungen ausgehärtet.
Die ausgehärteten Beschichtungen zeigen die in Tabelle 2 angegebenen Eigenschaften.

**Tabelle 2:**

| Beispiel II.1 | erfindungsgemäß (Polymercaptopolyamin) | Vergleich (DETA) |
|---|---|---|
| Viskosität (DIN 53018 T1/76) [mPa · s] | 5600 | 8650 |
| Gelierzeit nach TECAM bei 20 °C[min] | 35 | 15 |
| Staubtrockenzeit [h] bei 20 °C | 2.0 | > 30 |
| bei 5 °C | 2.5 | > 30 |
| Ausschwitzen bei 5 °C | nein | stark |
| Härte nach Persoz (ISO 1552) [s] bei 20 °C | | |
| nach 1 Tag | 358 | 340 |
| nach 1 Woche | 383 | 365 |
| nach 1 Monat | 394 | 355 |
| Härte nach Persoz (ISO 1552) [s] bei 5 °C | | |
| nach 1 Tag | 235 | 80 |
| nach 1 Woche | 267 | 220 |
| nach 1 Monat | 386 | 250 |

### II.2 Polymercaptopolyamin als Co-Härter im Gemisch mit anderen Polyaminhärtern

100 g eines flüssigen Bisphenol A-diglycidylether mit einem Epoxidgehalt von 5,25-5,4 val/kg werden mit 17 g eines handelsüblichen Aminhärters (DETA) und 4,2 g des Polymercaptopolyamins aus Beispiel 1.5 vermischt. Das Gemisch wird wie in Beispiel II.1 angegeben verarbeitet und ausgehärtet.
Zum Vergleich wird die oben angegebene Mischung ohne den Zusatz des Polymercaptopolyamins unter den gleichen Bedingungen ausgehärtet.
Die ausgeärteten Beschichtungen zeigen die in Tabelle 3 angegebenen Eigenschaften.

**Tabelle 3:**

| Beispiel 11.2 | erfindungsgemäß (Polymercaptopolyamin + DETA) | Vergleich (DETA) |
|---|---|---|
| Gelierzeit nach TECAM bei 20 °C [min] | 19 | 15 |
| Staubtrockenzeit [h] bei 20 °C/65 % rel. Luftfeuchtigkeit | 4 | >30 |
| Durchhärtungszeit [h) bei 20 °C/65 % rel. Luftfeuchtigkeit | 13 | >30 |
| Härte nach Persoz (ISO 1552) [s] | | |
| nach 1 d bei 20 °C | 310 | 195 |
| nach 7 d bei 20 °C | 349 | 229 |
| nach 1 d bei 5 °C | 73 (klebrig) | 24 (klebrig) |
| nach 7 d bei 5 °C | 148 (klebrig) | 39 (klebrig) |

### II.3 Polymercaptopolyamin als Härter für Epoxidharze

Das gemäß Beispiel I.26 hergestellte Polymercaptopolyamin wird mit den in Tabelle 4 angegebenen Epoxidharzen und weiteren Zusätzen gemischt und ausgehärtet.
Die Eigenschaften der Mischungen und der gehärteten Produkte sind ebenfalls in Tabelle 4 angegeben.

**Tabelle 4:**

| Beispiel | II.3.1 | II.3.2 | II.3.3 |
|---|---|---|---|
| Epoxid 9 [g] | 41 | | |
| Epoxid 8 [g] | | 37,4 | |
| Epoxid 6 [g] | | | 43,32 |
| Polymercaptopolyamin [g] | 29 | 32,6 | 26,68 |
| TiO₂ (Kronos 2310) [g] | 30 | 30 | 30 |
| Fließmittel BYK 300 [g] | 0,14 | 0,14 | 0,14 |
| Methylethylketon [g] | | | 8,8 |
| Viskosität (Epprecht Viskosimeter) bei 20 °C | | | |
| der frisch zubereiteten Mischung [mPa · s] | 5760 | 3520 | 2720 |
| nach 60 min [mPa · s] | >128000 | >128000 | 76800 |
| Härtung 12 Tage bei RT | | | |
| Härte nach Persoz [s] | 20 | 24 | 63 |
| Schlagverformung ¹⁾ (direkt impact) [cm · kg] | >160 | >30 | >60 |
| Schlagverformung (reverse impact) [cm · kg] | >80 | >20 | >20 |
| Erichsentiefungstest²⁾ [mm] | 10,5 | 8.9 | 9,3 |
| Acetontest | 2 | 2 | 1-2 |
| Härtung 30 min bei 80 °C und 12 d bei RT | | | |
| Härte nach Persoz [s] | 20 | 26 | 113 |
| Schlagverformung ¹⁾ (direkt impact) [cm · kg] | >160 | >30 | >60 |
| Schlagverformung (reverse impact) [cm · kg] | >80 | >10 | >30 |
| Erichsentiefungstest ²⁾ [mm] | 10,6 | 9.1 | 9,8 |
| Acetontest ³⁾ | 2 | 2 | 1 |

| | | | |
|---|---|---|---|
| ¹⁾ Die Schlagverformung (direkt impact) wird bestimmt, indem man einen Stempel mit einem Gewicht von 2 kg, an dessen Unterseite sich eine Kugel von 20 mm Durchmesser befindet, mit der Unterseite voraus aus bestimmter Höhe direkt auf die beschichtete Fläche fallen lässt. Der angegebene Wert ist das Produkt aus dem Gewicht des Stempels in kg und der Versuchshöhe in cm, bei der noch keine Beschädigung der Beschichtung feststellbar ist. Bei der Schlagverformung (reverse impact) wird der Stempel auf die der Beschichtung abgewandten Seite falllengelassen. | | | |
| ²⁾ nach DIN 53156 | | | |
| ³⁾ Nach DIN 53320.Die Probe wird 1min in Aceton gehalten. Das Ergebnis wird gemäß der folgenden fünfteiligen Skala beurteilt: 0 = unverändert; 1 = bremsend, nicht mit dem Fingernagel kratzbar; 2 = schwer kratzbar, evtl. Watte gefärbt; 3 = erweicht, leicht kratzbar; 4 = beginnende Ab- oder Auflösung; 5 = vollständige Auflösung. | | | |

## Patentansprüche

1. Verbindung der Formel la oder Ib, 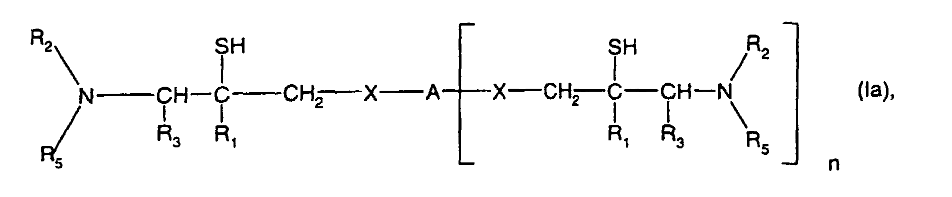 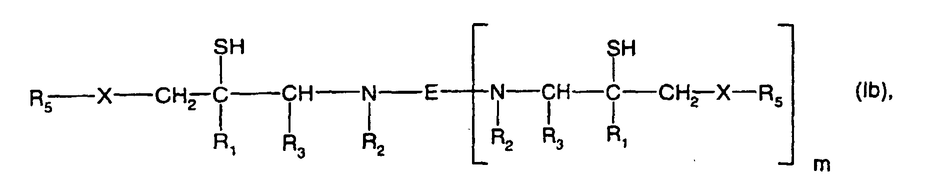 worin A einen (n + 1)-wertigen aliphatischen, cycloaliphatischen, araliphatischen oder aromatischen Rest darstellt und n eine ganze Zahl von 0 bis 5 ist,
E einen (m + 1)-wertigen aliphatischen, cycloaliphatischen, araliphatischen oder aromatischen Rest darstellt und m eine ganze Zahl von 0 bis 3 ist,
X für -O-, -COO-, oder -CHR₄- steht, wobei R₄ und R₃ zusammen eine Ethylengruppe bilden,
R₁ und R₂ unabhängig voneinander Wasserstoff oder Methyl bedeuten,
R₃ Wasserstoff bedeutet oder R₃ und R₄ zusammen eine Ethylengruppe bilden, und R₅ einen einwertigen aliphatischen, cycloaliphatischen, araliphatischen oder aromatischen Rest darstellt.

2. Verbindung der Formel la nach Anspruch 1, worin X -O- bedeutet und A für einen zweiwertigen Rest eines Bisphenols oder eines cycloaliphatischen Diols, für den Rest eines Phenol- oder Kresolnovolaks, für den zwei- bis vierwertigen Rest eines Isocyanat/Polyol-Addukts oder für den drei- bis sechswertigen Rest eines tri- bis hexafunktionellen aliphatischen Polyols steht.

3. Verbindung der Formel la nach Anspruch 1, worin X -O- bedeutet und A für einen zweiwertigen Rest der Formeln 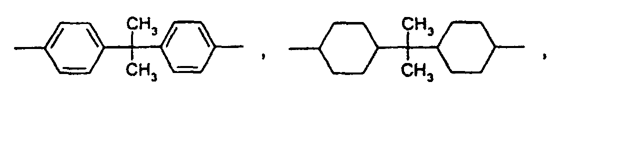 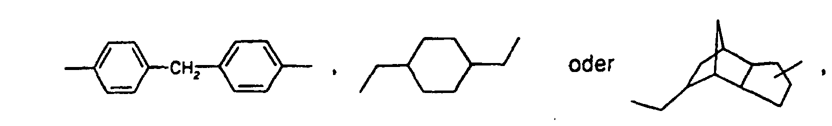 für den Rest eines Phenol- oder Kresolnovolaks, für einen dreiwertigen Rest der Formeln 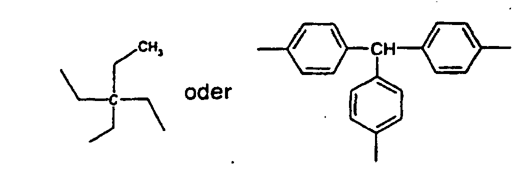 oder für den vierwertigen Rest der Formel 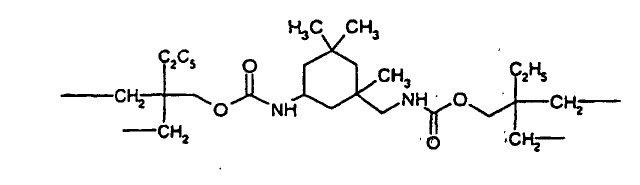 steht.

4. Verbindung der Formel la oder Ib nach Anspruch 1, worin R₅ unsubstituiertes oder mit einer oder mehreren Aminogruppen, Hydroxylgruppen, C₁-C₈-Alkoxygruppen oder Halogenatomen substituiertes C₁-C₂₀-Alkyl, C₅-C₁₂-Cycloalkyl, C₆-C₁₀-Aryl oder C₇-C₁₂-Aralkyl bedeutet.

5. Verbindung der Formel la oder Ib nach Anspruch 1, worin R₅ für C₂-C₁₀-Alkyl, C₂-C₁₀-Aminoalkyl, Phenyl, Benzyl, Cyclohexyl oder für einen Rest der Formel H₂N-Z-CH₂-NH- steht, worin Z einen zweiwertigen cycloaliphatischen, araliphatischen oder aromatischen Rest oder einen Rest der Formel -(CH₂CH₂NH)ₖ-CH₂- darstellt, worin k 2 oder 3 bedeutet.

6. Verbindung der Formel Ia oder Ib nach Anspruch 1, worin R₅ für n-Butyl, n-Octyl, Cyclohexyl, Benzyl, 2-Aminoethyl, 4-(Aminomethyl)pentyl, 5-Amino-2-meihylpentyl, 3-Dimethylaminopropyl, 3-Methylaminopropyl, 4-Aminocyclohexyl oder für einen Rest der Formeln -CH₂CH₂NHCH₂CH₂NH₂, 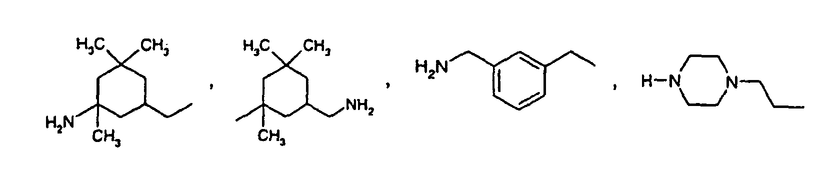 oder 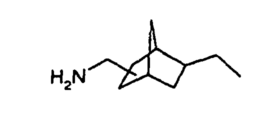 steht.

7. Verbindung der Formel Ia oder Ib nach Anspruch 1, worin X für O- steht und R₁ und R₃ Wasserstoff bedeuten.

8. Verfahren zur Herstellung von Verbindungen der Formel la gemäß Anspruch 1 durch Reaktion einer Verbindung der Formel IIa 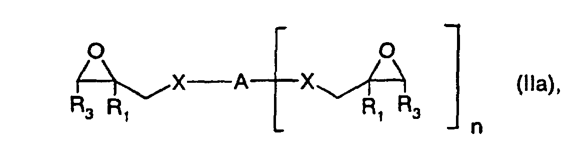 worin A, X, R₁, R₃ und n die in Anspruch 1 angegebene Bedeutung haben, mit Thioharnstoff oder einem Thiocyanat und anschließender Umsetzung des so erhaltenen Episulfids mit einem Amin der Formel R₅-NH-R₂, worin R₅ und R₂ die in Anspruch 1 angegebene Bedeutung haben.

9. Verfahren zur Herstellung von Verbindungen der Formel Ib gemäß Anspruch 1 durch Reaktion einer Verbindung der Formel IIb 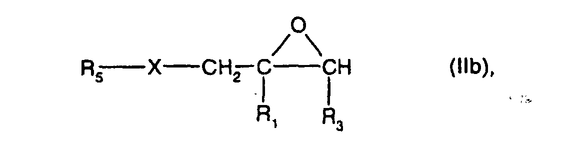 worin X, R₁, R₃ und R₅ die in Anspruch 1 angegebene Bedeutung haben,
mit Thioharnstoff oder einem Thiocyanat und anschließender Umsetzung des so erhaltenen Episulfids mit einem Polyamin der Formel E-(NHR₂)ₘ₊₁, worin E R₂ und m die in Anspruch 1 angegebene Bedeutung haben.

10. Zusammensetzung enthaltend
(A) ein Epoxidharz und
(B) eine Verbindung der Formel la oder Ib gemäß Anspruch 1.

11. Zusammensetzung nach Anspruch 10 enthaltend zusätzlich
(C) ein Polyamin.

12. Zusammensetzung nach Anspruch 10 oder 11 enthaltend die Komponenten B und gegebenfalls C in solchen Mengen, daß die Summe der Amin- und Mercaptanäquivalente 0,5 bis 2,0 Äquivalente, bezogen auf ein Epoxidäquivalent, beträgt.

13. Vernetzte Produkte erhältlich durch Härtung einer Zusammensetzung nach Anspruch 10.

14. Verwendung einer Zusammensetzung nach Anspruch 10 als Beschichtungsmittel, Klebstoff, Bindemittel für Verbundwerkstoffe oder Gießharz zur Herstellung von Formkörpern.

## Claims

1. A compound of formula la or Ib, 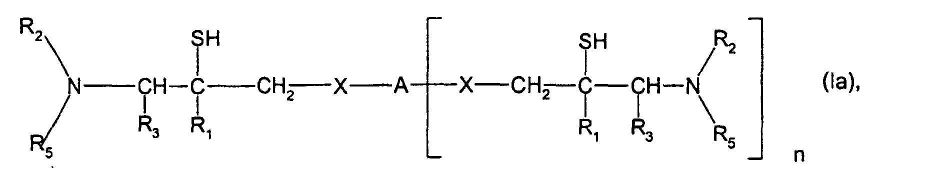 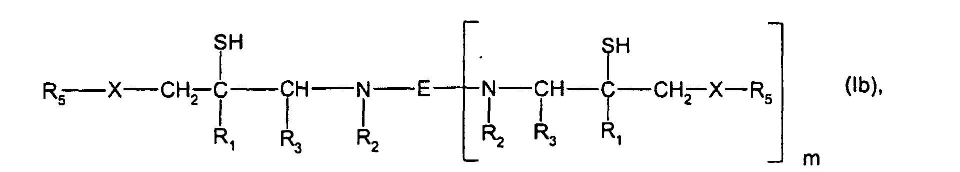 wherein A is an (n + 1)-valent aliphatic, cycloaliphatic, araliphatic or aromatic radical and n is an integer from 0 to 5,
E is an (m + 1)-valent aliphatic, cycloaliphatic, araliphatic or aromatic radical and m is an integer from 0 to 3,
X is -O-, -COO- or -CHR₄-, with R₄ and R₃ together forming an ethylene group,
R₁ and R₂ are, each independently of the other, hydrogen or methyl,
R₃ is hydrogen, or R₃ and R₄ together form an ethylene group,
and R₅ is a monovalent aliphatic, cycloaliphatic, araliphatic or aromatic radical.

2. A compound of formula la according to claim 1, wherein X is -O- and A is a bivalent radical of a bisphenol or of a cycloaliphatic diol, the radical of a phenol novolak or cresol novolak, the bi- to tetra-valent radical of an isocyanate/polyol adduct or the tri- to hexa-valent radical of a tri- to hexa-functional aliphatic polyol.

3. A compound of formula la according to claim 1, wherein X is -O- and A is a bivalent radical of formula 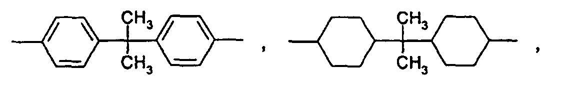 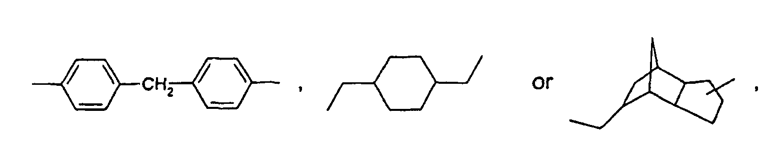 the radical of a phenol novolak or cresol novolak, a trivalent radical of formula 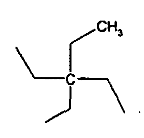 or 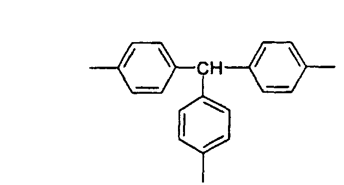 or the tetravalent radical of formula

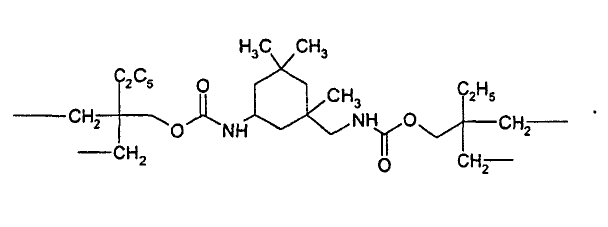

4. A compound of formula Ia or Ib according to claim 1, wherein R₅ is C₁-C₂₀alkyl, C₅-C₁₂-cycloalkyl, C₆-C₁₀aryl or C₇-C₁₂aralkyl, each of which is unsubstituted or substituted by one or more amino groups, hydroxyl groups, C₁-C₈alkoxy groups or halogen atoms.

5. A compound of formula la or Ib according to claim 1, wherein R₅ is C₂-C₁₀alkyl, C₂-C₁₀aminoalkyl, phenyl, benzyl, cyclohexyl or a radical of formula H₂N-Z-CH₂-NH-, wherein Z is a bivalent cycloaliphatic, araliphatic or aromatic radical or a radical of formula -(CH₂CH₂NH)ₖ-CH₂-, wherein k is 2 or 3.

6. A compound of formula la or Ib according to claim 1, wherein R₅ is n-butyl, n-octyl, cyclohexyl, benzyl, 2-aminoethyl, 4-(aminomethyl)pentyl, 5-amino-2-methylpentyl, 3-dimethylaminopropyl, 3-methylaminopropyl, 4-aminocyclohexyl or a radical of formula -CH₂CH₂NHCH₂CH₂NH₂, 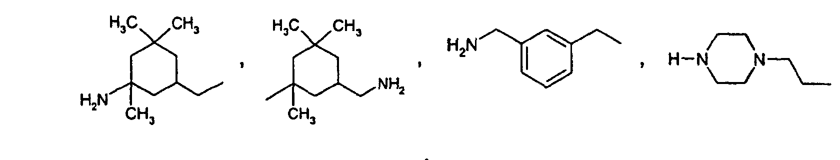 or

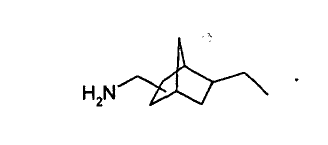

7. A compound of formula la or Ib according to claim 1, wherein X is O- and R₁ and R₃ are hydrogen.

8. A process for the preparation of a compound of formula la according to claim 1 by reacting a compound of formula IIa 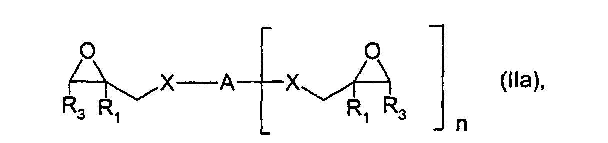 wherein A, X, R₁, R₃ and n are as defined in claim 1,
with thiourea or a thiocyanate and subsequently reacting the resulting episulfide with an amine of formula R₅-NH-R₂ wherein R₅ and R₂ are as defined in claim 1.

9. A process for the preparation of a compound of formula Ib according to claim 1 by reacting a compound of formula IIb 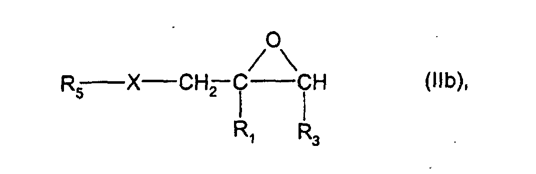 wherein X, R₁, R₃ and R₅ are as defined in claim 1,
with thiourea or a thiocyanate and subsequently reacting the resulting episulfide with a polyamine of formula E-(NHR₂)ₘ₊₁ wherein E, R₂ and m are as defined in claim 1.

10. A composition comprising
(A) an epoxy resin and
(B) a compound of formula la or Ib according to claim 1.

11. A composition according to claim 10 comprising, in addition,
(C) a polyamine.

12. A composition according to either claim 10 or claim 11 comprising component B and, where applicable, component C in such amounts that the sum of the amine and mercaptan equivalents is from 0.5 to 2.0 equivalents, based on one epoxy equivalent.

13. A cross-linked product obtainable by curing a composition according to claim 10.

14. Use of a composition according to claim 10 as coating composition, adhesive, bonding composition for composite materials or casting resin for the manufacture of mouldings.

## Revendications

1. Composé de formule Ia ou Ib, 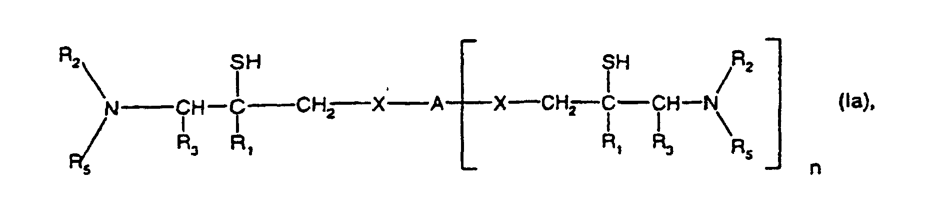 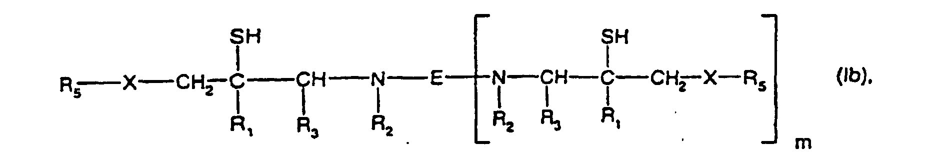 dans laquelle A représente un groupe aromatique, aliphatique, cycloaliphatique ou araliphatique valant (n + 1) et n représente un nombre entier de 0 à 5,
E représente un groupe aromatique, aliphatique, cycloaliphatique ou araliphatique valant (m + 1) et m représente un nombre entier de 0 à 3,
X représente -O-, -COO- ou -CHR₄-, R₄ et R₃ formant ensemble un groupe éthylène,
R₁ et R₂ représentent, indépendamment l'un de l'autre, un atome d'hydrogène ou un groupe méthyle,
R₃ représente un atome d'hydrogène ou R₃ et R₄ forment ensemble un groupe éthylène,
et R₅ représente un groupe aromatique, aliphatique, cycloaliphatique ou araliphatique monovalent.

2. Composé de formule la selon la revendication 1, où X représente -O- et A représente un reste bivalent d'un bisphénol ou d'un diol cycloaliphatique, le reste d'une novolaque phénol ou crésol , le resté bi- ou tëtra-valent d'un produit d'addition isocyanate/polyol ou le reste tri- à hexa-valent d'un polyol tri- à hexa-fonctionnel aliphatique.

3. Composé de formule la selon la revendication 1, où X représente -O- et A représente un reste bivalent de formule 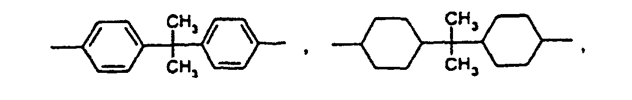 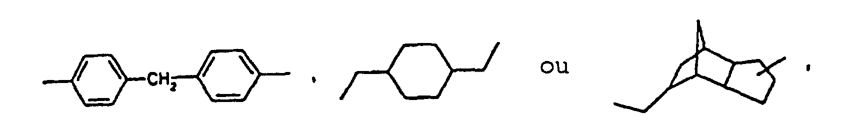 le reste d'une novolaque phénol ou crésol, le reste trivalent de formule 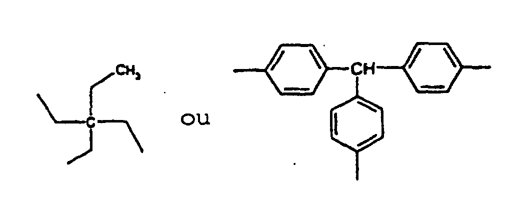 ou le reste tétravalent de formule

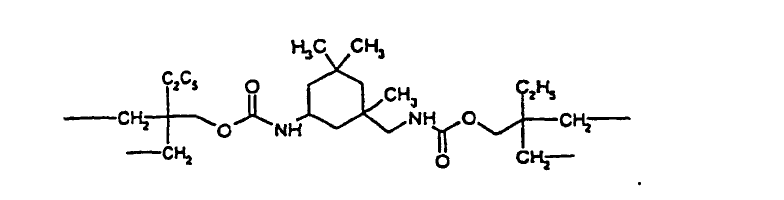

4. Composé de formule la ou Ib selon la revendication 1, où R₅ représente un groupe alkyle en C₁ à C₂₀, un groupe cycloalkyle en C₅ à C₁₂, aryle en C₆ à C₁₀ ou aralkyle en C₇ à C₁₂, non substitué ou substitué par un ou plusieurs groupes amino, hydroxyle, alcoxy en C₁ à C₈ ou halogéno.

5. Composé de formule la ou Ib selon la revendication 1, où R₅ représente un groupe alkyle en C₂ à C₁₀, aminoalkyle en C₂ à C₁₀, phényle, benzyle, cyclohexyle ou un groupe de formule H₂N-Z-CH₂-NH-, où Z représente un groupe aromatique, cycloaliphatique ou araliphatique bivalent ou un groupe de formule -(CH₂CH₂NH)ₖ-CH₂-, où k vaut 2 ou 3.

6. Composé de formule la ou Ib selon la revendication 1, où R₅ représente un groupe n-butyle, n-octyle, cyclohexyle, benzyle, 2-aminoéthyle, 4-(aminométhyl)pentyle, 5-amino-2-méthylpentyle, 3-diméthylaminopropyle, 3-méthylaminopropyle, 4-aminocyclohexyle ou un groupe de formule -CH₂CH₂NHCH₂CH₂NH₂, 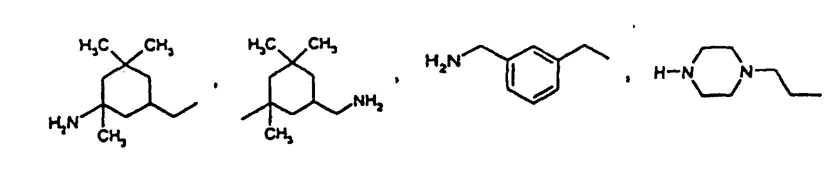 ou

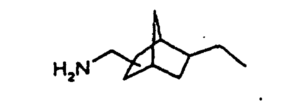

7. Composé de formule la ou Ib selon la revendication 1, où X représente O- et R₁ et R₃ représentent un atome d'hydrogène.

8. Procédé de production de composés de formule la selon la revendication 1, au moyen de la réaction d'un composé de formule IIa 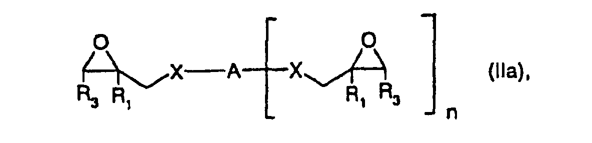 dans laquelle A, X, R₁, R₃ et n ont la signification indiquée dans la revendication 1, avec une thiourée ou un thiocyanate, suivie de la réaction de l'épisulfure ainsi obtenu avec une amine de formule R₅-NH-R₂, dans laquelle R₅ et R₂ ont la signification indiquée dans la revendication 1.

9. Procédé de production de composés de formule Ib selon la revendication 1, au moyen de la réaction d'un composé de formule IIb 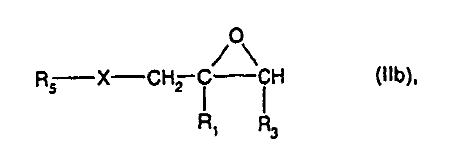 dans laquelle X, R₁, R₃ et R₅ ont la signification indiquée dans la revendication 1, avec une thiourée ou un thiocyanate, suivie de la réaction de l'épisulfure ainsi obtenu avec une polyamine de formule E-(NHR₂)ₘ₊₁, dans laquelle E, R₂ et m ont la signification indiquée dans la revendication 1.

10. Composition contenant
(A) une résine époxyde et
(B) un composé de formule la ou Ib selon la revendication 1.

11. Composition selon la revendication 10, contenant en plus
(C) une polyamine.

12. Composition selon la revendication 10 ou 11, contenant les composants B et éventuellement C dans des quantités telles que la somme des équivalents amine et mercaptan est de 0,5 à 2,0 équivalents par rapport à un équivalent époxyde.

13. Produits réticulés que l'on peut obtenir par durcissement d'une composition selon la revendication 10.

14. Utilisation d'une composition selon la revendication 10 en tant que matière de revêtement, adhésif, liant pour matériaux composites ou résine à couler pour la fabrication de corps moulés.
